# EUROPEAN PATENT SPECIFICATION

(11) **EP 0 823 852 B1**
(45) Date of publication and mention of the grant of the patent: **06.05.2004**
(21) Application number: 96909546.2
(22) Date of filing: 29.02.1996
(51) Int. Cl.: A61M 35/00, A61K 49/00

(54) **PREPACKAGED SWAB STICK FOR EPITHELIAL CANCER SCREENING**
ABGEPACKTE TUPFSTÄBCHEN ZUM NACHWEIS VON KREBS DES EPITHELS
BATONNET A TAMPON D'OUATE PRECONDITIONNE POUR LE DEPISTAGE DU CANCER EPITHELIAL

(43) Date of publication of application: 18.02.1998
(73) Proprietor: Zila, Inc., Phoenix, AZ 85014 (US)
(72) Inventor: HINES, Joseph, Paradise Valley, AZ 85253 (US)
(74) Representative: Wakerley, Helen Rachael
(86) International application number: PCT/US1996/002734
(87) International publication number: WO 1997/031675

(56) References cited:
- EP-A- 0 357 261
- US-A- 3 634 198
- US-A- 4 953 560
- US-A- 5 372 801
- US-A- 5 378 226
- DATABASE MEDLINE [Online] US NATIONAL LIBRARY OF MEDICINE (NLM), BETHESDA, MD, US LAU S K ET AL: "The cytological diagnosis of nasopharyngeal carcinoma using a silk swab stick." retrieved from STN Database accession no. 92144911 XP002131572 & CYTOPATHOLOGY, (1991) 2 (5) 239-46. ,

## Description

### BACKGROUND

This invention relates to improvements in screening patients for epithelial cancer.

In another respect, the invention relates to a prepackaged swabstick for use in screening patients to detect possible epithelial cancer.

More particularly, the invention pertains to a prepackaged swabstick for use in screening patients for epithelial cancer which can be used by para-professional medical personnel without highly specialized training.

In yet another respect, the invention pertains to a prepackaged swabstick for use in screening patients for epithelial cancer, which places liquid diagnostic reagents at the immediate disposal of clinical personnel, without requiring time-consuming or complicated mixing-storage-dispensing-measuring steps.

Various procedures are known for screening patients for epithelial cancer, especially for detecting suspected cancerous and precancerous sites on the mucosa such as the oral mucosa. Such procedures generally employ a dye which preferentially stains RNA-rich tissues which have been recognized as characteristic of cancerous and precancerous conditions. For example, U.S. Patent to *Mashberg* No. 4,321,251, discloses such a procedure. The procedure of *Mashberg* involves sequential washes, rinses and applications of water, dilute acetic acid and a solution of toluidine blue O dye, in the mouth to detect cancerous and precancerous conditions of the oral mucosa. This wash-rinse-application-rinse-wash procedure is then repeated after 10-14 days when a suspect site is detected, to reduce the number of false positives. Each repetition of the procedure involves a pre-rinse with acetic acid, two pre-rinses with water, a rinse with toluidine blue O dye, a post-rinse with acetic acid and a post-rinse with water:

It would be highly advantageous to screen all patients for epithelial cancer during routine office visits such as visits to dental offices for teeth cleaning, etc. However, methods of screening for epithelial cancer such as the *Mashberg* procedure have not achieved routine use, because the mixing, measuring and dispensing the solutions required is messy and too time consuming to be done for each patient by the dentist and such procedures require some training and experience beyond that normally possessed by para-professional assistants.

Further, the devices for delivering the liquid testing reagent to the suspected cancerous or precancerous sites are crude and cumbersome and generally not accepted by medical doctors and dentists. For example, present cancer screening procedures require that the reagent, for example toluidine blue, be administered by rinsing the entire mouth with the reagent or by applying the reagent directly to the suspected cancerous or precancerous site by using a saturated swab. Rinse applications require that the liquid reagent be poured from a larger container into a small cup to facilitate introducing the reagent into the mouth of a patient. Likewise, swab application requires that the absorbent swab be placed into a container containing the liquid reagent. The swab is then removed from the container and inserted into a patient's mouth to apply the reagent to the suspect cancerous or precancerous site. Each of these procedures is susceptible to spillage from the reagent container, reagent-containing cup or swab. Even a slight amount of spilled dye reagent can be extremely undesirable due to the inherent staining properties of the dye. US-A-5372801 discloses a dry stain composition which is dissolved in solvent prior to use.

Clinical swabs are utilized for many purposes such as cleaning wounds and applying anesthetics or disinfectants to body tissue. Prepackaged swabs are known. For example, the Clinipad Corporation manufactures a prepackaged swab sold under the mark Cliniswab. The Cliniswab kit includes a swab attached to the end of a plastic stick encased in a packet. The swab is of an absorbent material such as cotton and is saturated with an antiseptic or cleansing liquid such as iodine or isopropyl alcohol. EP-A-0357261 discloses prepackaged swabs used as applicators for liquids.

It would therefore be desirable to provide improvements in screening patients for epithelial cancer to promote routine screening as the adjunct to normal visits by patients to medical or dental offices for other reasons.

Yet another object of the invention is to provide apparatus which encourages such routine testing, by minimizing the time and complications which formally discouraged such routine testing by medical and dental professionals.

### SUMMARY

The present invention provides an improved apparatus for performing routine screening procedures to detect possible epithelial cancer. The improvement includes applying to suspected cancerous or precancerous sites a liquid testing reagent of preselected concentration. The present invention provides a prepackaged swabstick for performing routine screening procedures to detect possible epithelial cancer, said procedures including the step of applying to suspected cancerous or precancerous sites a liquid testing reagent, said prepackaged swabstick including a packet (7, 9) encasing an absorbent swab (3) disposed at an extremity of a handle (5), characterised in that: the absorbent swab (3) is saturated with the liquid testing reagent to permit direct dispensation of the reagent directly from its prepackaged form in according to the screening procedure, without further mixing or measuring steps.

The apparatus of the present invention includes a swab saturated with the liquid testing reagent attached to the end of a handle. The swabstick is encased in a packet to facilitate handling of the testing reagent and minimize spillage prior to application to the suspected cancerous or precancerous sites.

In accordance with the apparatus of the present invention, the prepackaged swabstick eliminates the mixing and measuring steps generally required of administering clinicians to prepare and apply a diagnostic reagent to a suspected cancerous or precancerous site on the oral mucosa.

An advantage of the present invention is simplified screening which will promote routine epithelial cancer screening during normal visits by the patients to medical or dental offices.

An additional advantage of the present invention is to eliminate the need for para-professional assistants by providing a simplified method of applying a testing reagent to suspected cancerous or precancerous sites of epithelial cancer.

Moreover, the present invention reduces or completely eliminates the damage to medical offices due to staining from the unexpected release or spillage of the liquid testing dye.

Other features and advantages of the present invention will be appreciated by those skilled in the art upon reading the detailed description which follows with reference to the attached drawing.

### BRIEF DESCRIPTION OF THE DRAWINGS

These and other features, aspects and advantages of the present invention, will become better understood to those skilled in the art from the following description, appended claims and accompanying drawing where:
Figure 1 depicts a prepackaged swabstick which is employed in accordance with principles of the present invention; and
Figure 2 depicts a preferred embodiment of the swabstick in accordance with principles of the present invention.

### DETAILED DESCRIPTION

While the present invention is susceptible of embodiment in various forms, there is shown in the drawing and will hereinafter be described, a presently preferred embodiment of the invention, with the understanding that the present disclosure is to be considered as; an exemplification of the invention, and I do not intend to limit the invention to the specific embodiments illustrated.

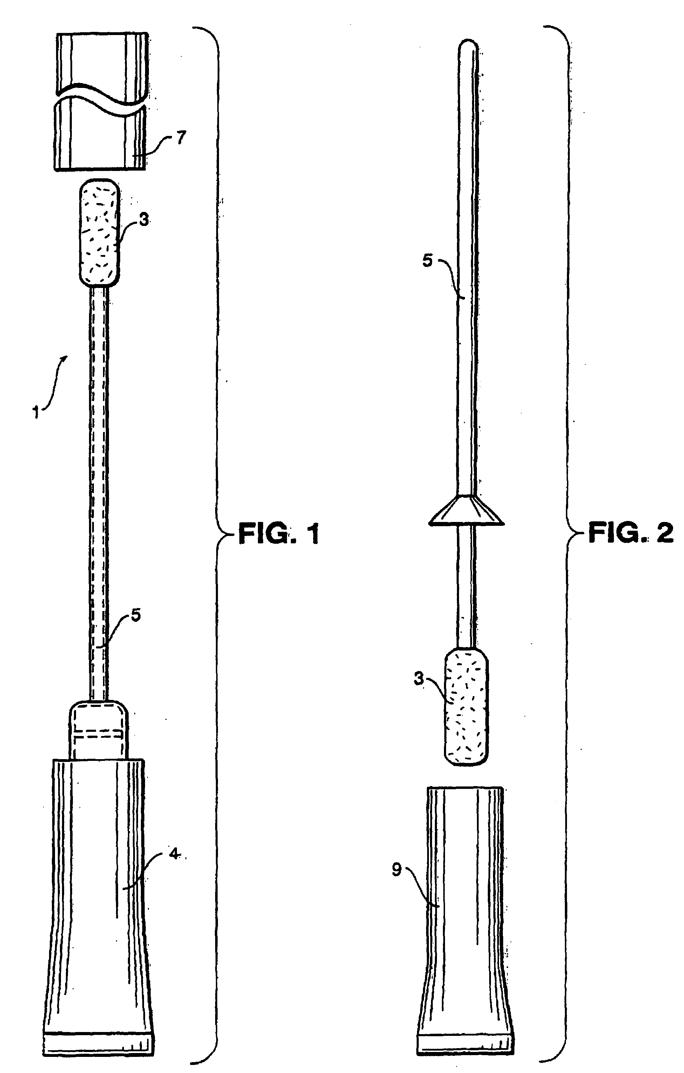

Referring to Figure 1, the prepackaged swabstick 1 includes a swab 3, a handle 5 and detachable cover 7. The swab 3 is of an absorbent material such as cotton and is affixed to the end of handle 5. As will be understood by those in the art, the handle could be manufactured from any of several biologically nonreactive materials such as plastic or treated wood, and can be produced in any number of configurations. For example, Figure 1 discloses handle 5 being configured as a cylinder with a concentric bore communicating with a container 4 for receipt and storage of the liquid testing reagent. The swab 3 is saturated with a liquid testing reagent (not shown) such as the dye toluidine blue O, for applying to suspected to cancerous or precancerous sites on the oral mucosa. Encasing the swab 3 and handle 5 is a cover 7 detachable from the container 4 to prevent the release or spillage of the liquid testing reagent prior to application to the suspected cancerous or precancerous sites.

In another embodiment of the prepackaged swabstick, depicted in Figure 2, the liquid reagent (not shown) is carried in a cylindrical container 9. In this configuration, the handle 5 is flexed to break away from the upper end of container 9 to allow withdrawal of the swab 3, soaked with the liquid reagent.

A prepackaged swabstick of the present invention is provided to the administering clinician. After removing the swabstick from its package, the swab 3, being saturated with a liquid testing reagent, is applied to suspected cancerous or precancerous sites on the patient's oral mucosa. Areas of the mucosa which are preferentially stained are recognized as characteristic of cancerous or precancerous conditions.

The liquid testing reagent is introduced into the mouth of a patient and the patient is advised to rinse and gargle. If a suspected site is detected after this first rinse application, the administering clinician applies the testing reagent to the suspected sites by application with the prepackaged swabstick. Preferably, application of the liquid testing reagent by administration with the prepackaged swabstick occurs 10 to 14 days after the initial rinse to reduce the likelihood of false positives.

The rinse procedure may be replaced by preferentially applying the liquid testing reagent to suspected cancerous or precancerous sites by application with the prepackaged swabstick of the present invention. By providing the administering clinician with a prepackaged swabstick for both the first screening administration and the sequential validating procedure, the messy and inconvenient step of rinsing the patient's mouth with the liquid testing reagent is thereby eliminated.

As will be apparent to those skilled in the art, my invention is not limited to a specific treatment method of specific solutions. Instead, it is generally applicable to any epithelial cancer detection procedure which employs a testing reagent, without limitation on the specific testing reagent employed.

Having described my invention in such terms as to enable those skilled in the art to understand and practice it, and having identified the presently preferred embodiments thereof, I claim:

## Claims

1. A prepackaged swabstick for performing routine screening procedures to detect possible epithelial cancer, said procedures including the step of applying to suspected cancerous or precancerous sites a liquid testing reagent, said prepackaged swabstick including a packet (7, 9) encasing an absorbent swab (3) disposed at an extremity of a handle (5),
**characterised in that**:
the absorbent swab (3) is saturated with the liquid testing reagent to permit direct dispensation of the reagent directly from its prepackaged form in accordance with the screening procedure, without further mixing or measuring steps.

2. A prepackaged swabstick according to claim 1 wherein the liquid testing reagent is a dye.

3. A prepackaged swabstick according to claim 1 wherein the liquid testing reagent is toluidine blue 0.

## Patentansprüche

1. Abgepacktes Tupferstäbchen für die Durchführung routinemäßiger Screening-Verfahren für den Nachweis von möglichem Epithelkrebs, wobei das genannte Verfahren den Schritt des Aufbringens eines flüssigen Testreagens auf mutmaßliche kanzeröse oder vorkanzeröse Stellen umfasst,
wobei das abgepackte Tupferstäbchen eine Verpackung (7, 9) aufweist, die einen absorptionsfähigen Tupfer (3) umgibt, der sich an einem äußeren Ende eines Griffs (5) befindet, **dadurch gekennzeichnet, dass**:
der absorptionsfähige Tupfer (3) mit dem flüssigen Testreagens getränkt ist, um eine direkte Dispensation des Reagens direkt aus seiner abgepackten Form gemäß dem Screening-Verfahren zu ermöglichen, ohne dass weitere Mischoder Messschritte notwendig sind.

2. Abgepacktes Tupferstäbchen gemäß Anspruch 1, wobei das flüssige Testreagens ein Farbstoff ist.

3. Abgepacktes Tupferstäbchen gemäß Anspruch 1, wobei das flüssige Testreagens Toluidinblau-O ist.

## Revendications

1. Tampon-tige préconditionné pour exécuter des procédures de dépistage de routine dans le but de détecter un cancer épithélial possible, lesdites procédures comprenant l'étape consistant à appliquer un réactif de test liquide aux sites cancéreux ou précancéreux soupçonnés, ledit tampon-tige préconditionné comprenant un étui (7, 9) enveloppant un tampon absorbant (3) disposé à une extrémité d'une poignée (5),
**caractérisé en ce que** :
le tampon absorbant (3) est saturé avec le réactif de test liquide afin de permettre la distribution directe du réactif directement de sa forme préconditionnée conformément à la procédure de dépistage, sans étape ultérieure de mélange ou de mesure.

2. Tampon-tige préconditionné selon la revendication 1, dans lequel le réactif de test liquide est un colorant.

3. Tampon-tige préconditionné selon la revendication 1, dans lequel le réactif de test liquide est du bleu de toluidine O.
